# EUROPEAN PATENT APPLICATION

(11) **EP 0 549 948 A1**
(43) Date of publication of application: **07.07.1993**
(21) Application number: 92121338.5
(22) Date of filing: 15.12.1992
(51) Int. Cl.: D21H 21/54, D21H 27/00, B65D 5/00

(54) **Article having a smooth nonabrasive antislip coating**

(30) Priority: 31.12.1991 US 815206; 31.12.1991 US 816367
(71) Applicant: KIMBERLY-CLARK CORPORATION, Neenah Wisconsin 54957-0349 (US)
(72) Inventor: Kronzer, Francis Joseph, Marietta, Georgia 30066 (US); Tyner, Theodore John, Woodstock, Georgia 30188 (US); Allison, John Patrick, Marietta, Georgia 30066 (US)
(74) Representative: Diehl, Hermann O. Th., Dr.

(57) **Abstract**

An article of manufacture consisting of a substrate having a smooth nonabrasive antislip coating on part or all of a surface thereof, which coating is composed of from about 80 to about 99 percent by weight, based on the weight of the coating, of a binder having a glass transition temperature no lower than about -30°C and from about 1 to about 20 percent by weight, based on the weight of the coating, of thermally expanded microbeads having particle sizes before expansion in the range of from about 5 to about 30 µm. The coating has a dry static coefficient of friction, when tested in accordance with ASTM Method 1894 against an aluminum plate having an anodized surface with a roughness of less than about 0.8 µm (32 microinches), of at least about 0.6; and a dry dynamic coefficient of friction, when tested in like manner, of at least about 0.8. The substrate can be paper, film, or a woven or nonwoven fabric. A preferred application is a dispensing container having the smooth nonabrasive antislip coating on part or all of the bottom surface thereof.

## Description

The present invention relates to an article of manufacture comprising a substrate having a smooth nonabrasive antislip coating on part or all of a surface thereof. The article according to a specific embodiment of the invention can be a dispensing container having a smooth nonabrasive antislip coating on the bottom surface thereof.

Antislip or non-skid coatings are, of course known. For example, non-skid surface compositions for paper products are disclosed in U.S. Patent No. 4,418,111 to Carstens. The composition consists of an aqueous suspension of colloidal silica and urea. The silica typically is present at a level of from approximately 1 to 5 percent by weight and has particle sizes in the range of 10 to 150 mµm. A urea/silica weight ratio between 0.10 and slightly greater than 3.00 is most effective. The composition is applied to the paper product by, for example, spraying.

An antiskid paper with enhanced friction retention is described in U.S. Patent No. 4,980,024 to Payne et al. According to this reference, superiority in retained slide angle of antiskid paper is achieved by spraying or otherwise coating the paper with a composition of matter consisting essentially of silica sol, glycerine, and an acrylamide homopolymer.

As illustrated by the foregoing two patents, antislip coatings for papers typically employ an inorganic material in the coating binder. Such coatings often are abrasive and, in fact, are closely related to such abrasive papers as sandpapers, emery cloths, and the like, as illustrated by the references which follow. U.S. Patent No. 2,899,288 to Barclay relates to a method of forming an abrasive sheet. Briefly, a cloth backing sheet fabricated of a thermoplastic material is passed through a heated zone in which one face of the sheet is temporarily softened, an abrasive material is applied evenly over the softened face, the abrasive is pressed into the softened sheet by means of a nip roll and simultaneously cooled.

U.S. Patent No. 3,166,388 to Riegger et al. relates to a sandpaper. The sandpaper comprises a reinforced paper backing, a barrier material, a layer of making varnish, and a layer of abrasive grits embedded in the making varnish. The barrier material may be in the form of a barrier layer of flexible material which only partly penetrates into one side of the backing, or it may be in the form of flexible rubbery solids which penetrate into and partially fill the voids in the paper backing throughout its thickness. Backings comprise a web or network of wood pulp fibers and a multiplicity of relatively flexible and tough thermoplastic reinforcing members distributed substantially throughout the web in bonding relation with the wood pulp fibers.

The commercial introduction of thermally expandable microspheres a number of years ago has led to a number of different uses, some examples of which are given below.

U.S. Patent No. 4,006,273 relates to washable and dry-cleanable raised printing on fabrics. Raised prints and graphic designs on fabrics which can safely and effectively be dry-cleaned and washed are provided by formulating a cross-linkable polymer printing medium comprising an adherent film-forming cross-linkable polymer binder in a liquid vehicle therefor about 1 to 45 weight percent thermally expandable microspheres, based on the weight of the binder, applying said medium to a fabric, heating at a temperature of about 82.2 ° to 121.1° C (180° to 250°F) to expand the microspheres and cross-link the polymer, and then curing for about 1 minute at a temperature of about 148.8 ° C (300°F). The microspheres can have diameters from about 0.5 to about 300 µm, preferably from about 3 to 50 µm, and most preferably from about 5 to 20 µm.

U.S. Patent No. 4,044,176 to Wolinski et al. describes graphic arts media which offer raised, three-dimensional effects. A basic medium is formulated of a colorant, film-forming binder, a solvent vehicle, and thermally expandable microspheres. The microspheres are treated to preclude or inhibit solvation in the solvent vehicle by coating with a compound which is a non-solvent for the microspheres but which preferentially wets the surface thereof. Allyl alcohols having about 3 to 5 carbon atoms in the alkyl chain are employed. The medium is selectively applied to a substrate, dried, and heated to expand the microspheres.

Japanese Published Application No. 90/76,735 relates to the manufacture of slightly rough sheets. Such sheets, useful as wall and floor coverings, leather substitutes, packaging sheets, etc., are prepared by coating thermoplastic sheets, completely or in patterns, with resins containing microencapsulated blowing agents (e.g., butane) and simultaneously expanding the microcapsules and softening the resins. In an example, flame-retardant paper was coated in patterns with an acrylic polymer-PVC blend containing microencapsulated blowing agents, coated with a PVC plastisol, and heated at 225° C to give a sheet with a sandy appearance.

It may be noted that microspheres which are not thermally expandable also are known. A few applications for such microspheres are described below.

A woven polyester-backed flexible coated abrasive having microballoons in the backsize is described in U.S. Patent No. 4,111,667 to Adams. The backsize is used with heavy-duty flexible coated products, particularly polyester-backed coated abrasive for use in making belts. The otherwise conventional backsize coating includes from 2 to 10 percent by weight of hollow microspheres. The coating is applied on the reverse or nonabrasive side of a woven polyester backing. The microspheres (or microbeads or microballoons) are hollow spheres of resin or glass having a diameter in the range of between 5 and 125 µm.

U.S. Patent No. 4,543,106 to Parekh relates to a coated abrasive product containing hollow microspheres beneath the abrasive grain. The product comprises a fabric backing, a layer of abrasive grain, and at least one layer of resin between the backing and the abrasive grain. Hollow microspheres are present and at least partly and usually entirely embedded in the resin layer. In general, the hollow microspheres comprise hollow spherical bodies which may be of glass or plastic materials such as a phenolic resin, which have diameters from about 5 to about 500 µm and an average diameter of from about 25 to about 125 µm. The microspheres generally have a shell thickness which averages from about 5 to about 20 percent of the diameter of the microspheres. The microspheres usually are incorporated into the resin layer in an amount of from about 5 to about 20 percent by weight of the resin layer.

A reference which does not fit in any of the foregoing categories is included for the sake of completeness. That reference is U.S. Patent No. 5,001,106 to Egashira et al., which relates to an image-receiving sheet. Such sheet comprises a base sheet and a receiving layer, provided on one surface of the base sheet, for receiving a dye or pigment migrating from a heat transfer sheet. The base sheet comprises one or two or more layers, with at least one layer having a porous or foamed structure. A layer having a porous or foamed structure can be obtained by such methods as: (a) stretching a film prepared from a thermoplastic resin and containing fine inorganic or organic particles, (b) extruding an organic solvent solution of a synthetic resin into a coagulating bath, and (c) extruding a resin together with a foaming agent. Under certain circumstances, it is desireable to roughen at least a part of both front and back surfaces of the image-receiving sheet, e.g., the non-image portion of the receiving surface or the back surface of the image-receiving sheet, by imparting fine unevenness thereto.

References relating to the microbeads themselves include those described below.

U.S. Patent No. 3,615,972 to Morehouse, Jr. et al. describes expansible thermoplastic polymer particles containing volatile fluid foaming agent and a method of foaming the same. Thermoplastic microspheres are prepared which encapsulate a liquid blowing agent. Heating of the microspheres causes expansion. The microspheres are useful for coatings, moldings, plastic smoke, etc.

Polymer foam compositions are described in U.S. Patent No. 3,864,181 to Wolinski et al. The patent describes a composition and method for forming foamed polymers. The composition comprises a dispersion of microspheres in a solution of the polymer in a solvent. The compositions are applied to a substrate, dried, and heated to expand the microspheres, thus forming a foamed polymer. The particular surface characteristics of foamed polymers are stated to have been utilized in non-skid coatings for carpets, rugs, bathtub mats, flooring, coat hangers, handles for tools and athletic equipment, and the like.

U.S. Patent No. 4,722,943 to Melber et al. relates to a composition and process for drying and expanding microspheres. Microsphere wet cake is mixed with a processing aid effective to prevent agglomeration and surface bonding of the microspheres, and thereafter removing water by drying with continuous mixing, optionally also under reduced pressure, i.e., vacuum drying. By the control of the application of heat and balancing temperature and the mixing, and optionally also the reduced pressure, it is possible to also control expansion of the microspheres from substantially none to substantially theoretical limits of expansion. Suitable processing aids include, by way of example, dry inorganic pigments or filler materials and the like, and related organic materials. See also U.S. Patent Nos. 4,829,094 to Melber et al. and 4,843,104 to Melber et al.

Notwithstanding the foregoing, there still is a need for an antislip coating which is smooth and nonabrasive, especially for papers, films, and fabrics.

It therefore is an object of the present invention to provide an article of manufacture comprising a substrate having a smooth nonabrasive antislip coating on part or all of a surface thereof. This object is solved by the article of independent claim 1.

It also is an aspect of the present invention to provide an article of manufacture which comprises a paper having a first surface and a second surface, in which said first surface has a smooth nonabrasive, antislip coating adjacent to and contiguous with said first surface.

It is a further aspect of the present invention to provide a dispensing container having a smooth nonabrasive antislip coating on part or all of the bottom surface thereof.

Further advantageous features, aspects, and details of the invention are evident from the dependent claims, the description and examples.

The antislip property of the coating is obtained through incorporation in the coating binder of thermally expandable microbeads.

Accordingly, the present invention according to a first aspect provides an article of manufacture comprising a substrate having a smooth nonabrasive antislip coating on part or all of a surface thereof, which coating:
A. is comprised of from about 80 to about 99 percent by dry weight, based on the dry weight of the coating, of a binder having a glass transition temperature no lower than about -30°C and from about 1 to about 20 percent by dry weight, based on the dry weight of the coating, of thermally expanded microbeads having particle sizes before expansion in the range of from about 5 to about 30 µm;
B. has a dry static coefficient of friction, when tested in accordance with ASTM Method 1894 against an aluminum plate having an anodized surface with a roughness of less than about 0.8 µm (about 32 microinches), of at least about 0.6; and
C. has a dry dynamic coefficient of friction, when tested in accordance with ASTM Method 1894 against an aluminum plate having an anodized surface with a roughness of less than about 0.8 µm (about 32 microinches), of at least about 0.8;
in which said substrate is selected from the group consisting of papers, films, and woven and nonwoven fabrics.

The present invention according to a further aspect also provides an article of manufacture which comprises a paper having a first surface and a second surface, in which said first surface has a smooth nonabrasive, antislip coating adjacent to and contiguous with said first surface, which coating:
A. is comprised of from about 80 to about 99 percent by dry weight, based on the dry weight of the coating, of a binder having a glass transition temperature no lower than about -30°C and from about 1 to about 20 percent by dry weight, based on the dry weight of the coating, of thermally expanded microbeads having particle sizes before expansion in the range of from about 5 to about 30 µm;
B. has a dry static coefficient of friction, when tested in accordance with ASTM Method 1894 against an aluminum plate having an anodized surface with a roughness of less than about 0.8 µm (about 32 microinches), of at least about 0.6; and
C. has a dry dynamic coefficient of friction, when tested in accordance with ASTM Method 1894 against an aluminum plate having an anodized surface with a roughness of less than about 0.8 µm (about 32 microinches), of at least about 0.8.

In preferred embodiments, the coating also (a) has a wet static coefficient of friction, when tested in accordance with ASTM Method 1894 against an aluminum plate having an anodized surface with a roughness of less than about 0.8 µm (about 32 microinches), of at least about 0.6; and (b) has a wet dynamic coefficient of friction, when tested in accordance with ASTM Method 1894 against an aluminum plate having an anodized surface with a roughness of less than about 0.8 µm (about 32 microinches), of at least about 0.7.

The present invention according to a further aspect also provides a dispensing container having a smooth nonabrasive antislip coating on part or all of the bottom surface thereof, which coating:
A. is comprised of from about 80 to about 99 percent dry by weight, based on the dry weight of the coating, of a binder having a glass transition temperature no lower than about -30°C and from about 1 to about 20 percent by dry weight, based on the dry weight of the coating, of thermally expanded microbeads having particle sizes before expansion in the range of from about 5 to about 30 µm;
B. has a dry static coefficient of friction, when tested in accordance with ASTM Method 1894 against an aluminum plate having an anodized surface with a roughness of less than about 0.8 µm (about 32 microinches), of at least about 0.6; and
C. has a dry dynamic coefficient of friction, when tested in accordance with ASTM Method 1894 against an aluminum plate having an anodized surface with a roughness of less than about 0.8 µm (about 32 microinches), of at least about 0.8.

The unique smooth and nonabrasive coating employed permits placing the containers on any surface, including furniture.

### Detailed Description of the Invention

As use herein, the term "smooth" refers to the absence of sharp or angular particles in the nonabrasive antislip coating of the present invention. Rather, such coating contains smooth, e.g., spherical, microspheres. The term also refers to the feel of the surface when touched, i.e., to the tactile characteristics of the coating. That is, the coating feels "smooth" to the touch.

In general, the substrate can be any paper, film, or fabric. The fabric can be either woven or nonwoven, although a nonwoven fabric is preferred. The material from which a film or fabric is made is not known to be critical. The nonwoven webs include any of the known nonwoven webs, including, but not limited to, meltblown webs, spunbonded webs, coformed webs, bonded carded webs, laminates of two or more of such webs with or without additional layers, and the like.

As a practical matter, films and the preferred nonwoven fabrics are prepared from thermoplastic polymers. Examples of thermoplastic polymers include, by way of illustration only, end-capped polyacetals, such as poly(oxymethylene) or polyformaldehyde, poly(trichloroacetaldehyde), poly(n-valeraldehyde), poly(acetaldehyde), poly(propionaldehyde), and the like; acrylic polymers, such as polyacrylamide, poly(acrylic acid), poly(methacrylic acid), poly(ethyl acrylate), poly(methyl methacrylate), and the like; fluorocarbon polymers, such as poly(tetrafluoroethylene), perfluorinated ethylene-propylene copolymers, ethylene-tetrafluoroethylene copolymers, poly(chlorotrifluoroethylene), ethylene-chlorotrifluoroethylene copolymers, poly(vinylidene fluoride), poly(vinyl fluoride), and the like; polyamides, such as poly(6-aminocaproic acid) or poly(ε-caprolactam), poly(hexamethylene adipamide), poly(hexamethylene sebacamide), poly(11-aminoundecanoic acid), and the like; polyaramides, such as poly(imino-1,3-phenyleneiminoisophthaloyl) or poly(m-phenylene isophthalamide), and the like; parylenes, such as poly-p-xylylene, poly(chloro-p-xylylene), and the like; polyaryl ethers, such as poly(oxy-2,6-dimethyl-1,4-phenylene) or poly(p-phenylene oxide), and the like; polyaryl sulfones, such as poly(oxy-1,4-phenylenesulfonyl-1,4-phenyleneoxy-1,4-phenylene-isopropylidene-1,4-phenylene), poly(sulfonyl-1,4-phenyleneoxy-1,4-phenylenesulfonyl-4,4'-biphenylene), and the like; polycarbonates, such as poly(bisphenol A) or poly(carbonyldioxy-1,4-phenyleneisopropylidene-1,4-phenylene), and the like; polyesters, such as poly(ethylene terephthalate), poly(tetramethylene terephthalate), poly(cyclohexylene-1,4-dimethylene terephthalate) or poly(oxymethylene-1,4-cyclohexylenemethyleneoxyterephthaloyl), and the like; polyaryl sulfides, such as poly(p-phenylene sulfide) or poly(thio-1,4-phenylene), and the like; polyimides, such as poly(pyromellitimido-1,4-phenylene), and the like; polyolefins, such as polyethylene, polypropylene, poly(1-butene), poly(2-butene), poly(1-pentene), poly(2-pentene), poly(3-methyl-1-pentene), poly(4-methyl-1-pentene), 1,2-poly-1,3-butadiene, 1,4-poly-1,3-butadiene, polyisoprene, polychloroprene, polyacrylonitrile, poly(vinyl acetate), poly(vinylidene chloride), polystyrene, and the like; copolymers of the foregoing, such as acrylonitrile-butadiene-styrene (ABS) copolymers, and the like; and the like.

Thermoplastic polyolefins are preferred and include polyethylene, polypropylene, poly(1-butene), poly(2-butene), poly(1-pentene), poly(2-pentene), poly(3-methyl-1-pentene), poly(4-methyl-1-pentene), 1,2-poly-1,3-butadiene, 1,4-poly-1,3-butadiene, polyisoprene, polychloroprene, polyacrylonitrile, poly(vinyl acetate), poly(vinylidene chloride), polystyrene, and the like.

The more preferred polyolefins are those which contain only hydrogen and carbon atoms and which are prepared by the addition polymerization of one or more unsaturated monomers. Examples of such polyolefins include, among others, polyethylene, polypropylene, poly(1-butene), poly(2-butene), poly(1-pentene), poly(2-pentene), poly(3-methyl-1-pentene), poly(4-methyl-1-pentene), 1,2-poly-1,3-butadiene, 1,4-poly-1,3-butadiene, polyisoprene, polystyrene, and the like. In addition, such term is meant to include blends of two or more polyolefins and random and block copolymers prepared from two or more different unsaturated monomers. Because of their commercial importance, the most preferred polyolefins are polyethylene and polypropylene.

The most preferred substrates are papers and nonwoven fabrics prepared from thermoplastic polyolefins, especially meltblown and spunbonded nonwoven webs.

When the substrate is a paper, the nature of the paper is not known to be critical, provided it has sufficient strength for handling, coating, sheeting, and/or other operations associated with its manufacture. In preferred embodiments, the base sheet will be a latex-impregnated paper. By way of illustration, a preferred paper is a water leaf sheet of wood pulp fibers impregnated with a reactive acrylic polymer latex such as Rhoplex® B-15 (Rohm and Haas Company, Philadelphia, Pennsylvania). However, any of a number of other latexes can be used, if desired, some examples of which are summarized in Table I, below.

**Table I**

| Suitable Latexes for Base Sheet | |
|---|---|
| Polymer Type | Product Identification |
| Polyacrylates | Hycar® 26083, 26084, 26120, 26104, 26106, 26322 B. F. Goodrich Company Cleveland, Ohio |
| | Rhoplex® HA-8, HA-12, NW-1715 Rohm and Haas Company Philadelphia, Pennsylvania |
| | Carboset® XL-52 B. F. Goodrich Company Cleveland, Ohio |
| Styrene-butadiene copolymers | Butofan® 4262 BASF Corporation Sarnia, Ontario, Canada |
| | DL-219, DL-283 Dow Chemical Company Midland, Michigan |
| Ethylene-vinyl acetate copolymers | Dur-O-Set® E-666, E-646, E-669 National Starch & Chemical Co. Bridgewater, New Jersey |
| Nitrile rubbers | Hycar® 1572, 1577, 1570 x 55 B. F. Goodrich Company Cleveland, Ohio |
| Poly(vinyl chloride) | Geon® 552 B. F. Goodrich Company Cleveland, Ohio |
| Poly(vinyl acetate) | Vinac® XX-210 Air Products and Chemicals, Inc. Napierville, Illinois |
| Ethylene-acrylate copolymers | Mitchem® Prime 4990 Michelman, Inc. Cincinnati, Ohio |
| | Adcote® 56220 Morton Thiokol, Inc. Chicago, Illinois |

The impregnating dispersion typically also will contain clay and a delustrant such as titanium dioxide. Typical amounts of these two materials are 16 parts and 4 parts, respectively, per 100 parts of polymer on a dry weight basis. An especially preferred base sheet has a basis weight of 50 g/m² (13.3 lbs/1300 ft²) before impregnation. The impregnated paper preferably contains 18 parts impregnating solids per 100 parts fiber by weight, and has a basis weight of 58 g/m² (15.6 lbs/1300 ft²), both on a dry weight basis. A suitable caliper is 97 ± 8 µm (3.8 mils ± 0.3 mil).

Such a paper is readily prepared by methods which are well known to those having ordinary skill in the art. In addition, paper-impregnating techniques also are well known to those having ordinary skill in the art. Typically, a paper is exposed to an excess of impregnating dispersion, run through a nip, and dried.

Turning now to the smooth antislip coating, such coating, regardless of the substrate, is comprised of from about 80 to about 99 percent by dry weight, based on the dry weight of the coating, of a binder having a glass transition temperature no lower than about -30°C and from about 1 to about 20 percent by dry weight, based on the dry weight of the coating, of thermally expanded microbeads having particle sizes before expansion in the range of from about 5 to about 30 µm. In addition, such coating must have a dry static coefficient of friction, when tested in accordance with ASTM Method 1894 against an aluminum plate having an anodized surface with a roughness of less than about 0.8 µm (about 32 microinches), of at least about 0.6, and a dry dynamic coefficient of friction, when similarly tested, of at least about 0.8.

In certain preferred embodiments, particularly when the substrate will be used or exposed to a wet environment, the coating will have a wet static coefficient of friction, when tested in accordance with ASTM Method 1894 against an aluminum plate having an anodized surface with a roughness of less than about 0.8 µm (about 32 microinches), of at least about 0.6, and a wet dynamic coefficient of friction, when tested in a similar manner, of at least about 0.7.

Generally, the binder can be any of the known binders which commonly are used as the basis of coatings on paper and other substrates, provided the binder has a glass transition temperature no lower than about -30°C. It should be noted that binders having glass transition temperatures below ambient temperatures but no lower than about -30°C can yield coatings which are somewhat tacky at ambient temperatures. Although such binders still can be used, some applications may require the use of a release coating or a release paper in order to prevent the coating from sticking or adhering to the reverse side of the substrate, particularly when the substrate is to be manufactured, stored, and/or transported in roll form. However, the use of a release coating or a release paper can be obviated by using binders having glass transition temperatures no lower than about 15°C, preferably no lower than about 25°C.

The binder in general will constitute from about 80 to about 99 percent by dry weight, based on the dry weight of the coating, of the coating. As used herein, however, the term "binder" is meant to be sufficiently broad to include minor amounts of other materials, such as dyes, colorants, pigments, plasticizers, flow agents, antistatic agents, extenders, water repellents, surfactants, viscosity control agents, dispersing aids, and the like. Preferably, the amount of binder present will be in the range of from about 85 to about 95 percent by dry weight, based on the dry weight of the coating. The most preferred amount of binder is from about 88 to about 92 percent by dry weight.

The coating also contains from about 1 to about 20 percent by dry weight, based on the dry weight of the coating, of thermally expanded microbeads. The amount of such microbeads preferably is in the range of from about 5 to about 15 percent by dry weight, and most preferably is in the range of from about 8 to about 12 percent by dry weight.

In general, the thermally expanded microbeads can be any of the commercially available microbeads, such as those sold under the Expancel^{R} trademark by Nobel Industries Sweden, Sundsvall, Sweden, and those sold under the Foamcoat^{R} trademark by Pierce & Stevens Corporation, Buffalo, New York. While any of the various types of thermally expandable microbeads can be employed, the more heatresistant microbeads are preferred.

The level of coating on a given substrate typically can range from about 3.7 to about 11.5 g/m². The preferred range is from about 5.6 to about 9.4 g/m². It is neither necessary nor required, though, that the coating cover all of a surface of a particular substrate. When the substrate is a paper which will serve as the backing for an abrasive layer, the coating typically will cover all of one surface. When the substrate is a nonwoven web or a laminate of two or more nonwoven webs intended for use as a surgical drape or a shoe cover, the coating typically will be applied only to a portion of the target surface thereof.

As already stated, the coating must have a dry static coefficient of friction, when tested in accordance with ASTM Method 1894 against an aluminum plate having an anodized surface with a roughness of less than about 0.8 µm (less than about 32 microinches), of at least about 0.6, and a dry dynamic coefficient of friction, when similarly tested, of at least about 0.8. Moreover, in certain preferred embodiments, particularly when the substrate will be used or exposed to a wet environment, the coating will have a wet static coefficient of friction, when tested in like manner, of at least about 0.6, and a wet dynamic coefficient of friction, when tested in a similar manner, of at least about 0.7.

Coefficients of friction were determined in accordance with ASTM Method 1894 with a Model 1122 Instron Testing Machine (Instron Corporation, Canton, Massachusetts). Each sample tested was a 6.4 cm (2.5 inch) square. Dry samples of coated substrates were conditioned for 24 hours at 50 percent relative humidity and 22°C. Wet samples were soaked in 2 g of distilled water for 48 hours. Before testing, the water on the bottom surface, i.e., the surface not having the smooth nonabrasive antislip coating of the present invention, was removed by gentle blotting. The surface used to determine the coefficients of friction was an aluminum plate supplied by Instron having a clear anodized surface with a roughness of less than about 0.8 µm (about 32 microinches).

A preferred application for the smooth nonabrasive antislip coating of the present invention is a dispensing container having a smooth nonabrasive antislip coating on part or all of the bottom surface thereof.

The term "dispensing container" is meant to include any dispensing container which is intended to be placed on an essentially horizontal surface and remain essentially portable or moveable. Thus, the term excludes wall-mounted containers and any other dispensing containers which are mounted or otherwise fixed in a stationary position. The dispensing container most often will be a free-standing tissue or wipe dispenser.

The term "bottom surface" refers to the bottom, external surface of the dispensing container, i.e., the surface of the dispensing container which comes in contact with the surface upon which the dispensing container is placed.

The material from which the dispensing container is made is not known to be critical. As a practical matter, however, the container most often will be constructed from a cellulosic material, such as paper or cardboard.

The smooth nonabrasive antislip coating can be applied directly to the bottom surface of the dispensing container either during or after its manufacture. Alternatively, the coating can be applied to the first surface of a paper substrate having a contact adhesive protected by a release paper on the second surface. In this case, the coated paper can be applied to the dispensing container as part of the manufacturing process or by the consumer. Because a coated paper is more easily tested, such a paper was the focus of the work described hereinafter in one of the examples.

The percentage of the bottom surface to be coated is not known to be critical. Of course, the maximum antislip effect will be achieved when all of the bottom surface is coated. Coating only part of the bottom surface in many cases will give adequate antislip characteristics to the container, especially when heavier containers are involved. Conversely, small containers which generally are lighter preferably will have coating on the entire bottom surface.

When the coating is applied to a cellulosic sheet, the nature of the cellulosic sheet is not known to be critical, provided it has sufficient strength for handling, coating, sheeting, and/or other operations associated with its manufacture. In preferred embodiments, the sheet will be a latex-impregnated paper as already described.

The present invention is further illustrated by the examples which follow. In the examples, all parts are parts on a dry weight basis, unless stated otherwise.

### Examples 1-24

### Preparation of Coated Papers

Because of the convenience in handling, coating, and testing papers, all of the examples employed paper substrates. Three different papers were employed and are described below.

### Paper A

Paper A was a standard latex-impregnated paper. The basis weight of the paper before impregnation was 52.6 g/m². The latex saturant consisted of 100 parts of Butofan® 4262, a styrene-butadiene rubber (BASF Corporation, Sarnia, Ontario, Canada), 30 parts of clay, 2 parts of a colloidal stabilizer, 1 part of a water repellant, and 14 parts of a colorant (the same colorant was used in all saturant and antislip coating dispersions used in the examples). The saturant level in the paper on a dry weight basis was 26 parts per 100 parts of wood pulp fiber.

A barrier coating was applied to one surface of the paper (referred to herein for convenience as the second surface). The barrier coating was a dispersion which consisted of, on a dry weight basis, 100 parts of Styronal® 4574, also a styrene-butadiene rubber (BASF, Sarnia, Ontario, Canada), 57.5 parts of Hycar® 2600x106, an acrylic polymer (B. F. Goodrich Company, Cleveland, Ohio), and 30 parts of clay. The barrier coating latex was applied with a Meyer rod to provide a coating weight of 15-17 g/m².

### Paper B

This paper also was a standard latex-impregnated paper. The basis weight of the paper before impregnation was 52.6 g/m². The latex saturant was the same as that used for Paper A, except that the colorant level was 10. The saturant level in the paper on a dry weight basis was 38 parts per 100 parts of wood pulp fiber. A barrier was applied to the second surface as described for Paper A.

### Paper C

The paper was a standard latex-impregnated paper having a basis weight before impregnation of 52.6 g/m². The latex saturant consisted of 100 parts of Hycar® 1562, an acrylonitrile-butadiene rubber (B. F. Goodrich Company, Cleveland, Ohio), 4 parts of a phenolic resin, 30 parts of clay, 1.5 parts of a water repellant, and 9.28 parts of a colorant. The saturant level in the paper on a dry weight basis was 38 parts per 100 parts of wood pulp fiber. The paper had the barrier coating of Paper A on the second surface.

With the exception of Example 1 in which the first surface of the paper substrate was uncoated, the first surface of the paper substrate was coated with an antislip or control coating by means of a Meyer rod. The coating was applied in each case at a level of 8.3-9.4 g/m², on a dry weight basis, except for Examples 20-22, inclusive. The basis weights of the coatings employed in Examples 20-22, inclusive, were 5.6, 8.5, and 16.2 g/m², respectively. Each antislip coating of the present invention was dried for one minute at about 107°C and then was heated for 10-15 seconds at 165°C to expand the microbeads. Both the drying and expanding steps were carried out in a forced air oven. Coatings not containing thermally expandable microbeads were simply dried at 107°C. The compositions of the dispersions, on a dry weight basis, which were employed to prepare the various antislip and control coatings tested are summarized in Tables 2-5, inclusive. In general, the dispersions contained about 45 percent by weight solids, based on the total weight of the dispersion, with the remainder being water.

**Table 2**

| **Summary of Dispersion Compositions for Coatings A-F** | | | | | | |
|---|---|---|---|---|---|---|
| Component | Parts by Dry Weight in Coating | | | | | |
| | A | B | C | D | E | F |
| Rhoplex® HA16 | 100 | --- | --- | 100 | --- | --- |
| Styronal® ND846 | --- | 100 | --- | --- | --- | --- |
| Butofan® 4262 | --- | 50 | --- | --- | --- | --- |
| Rhoplex® 8-15 | --- | --- | --- | --- | 100 | --- |
| Michem® 4983 | --- | --- | --- | --- | --- | 100 |
| Styronal® 4574 | --- | --- | 100 | --- | --- | --- |
| Diatomaceous earth | 47 | 55.6 | --- | --- | --- | --- |
| Clay | 57 | --- | --- | --- | --- | --- |
| Water repellant | --- | 11.35 | --- | --- | --- | --- |
| Expancel® 051WU | --- | --- | 15 | 15 | 15 | 15 |
| Colorant | 3 | 3 | 3 | 3 | 3 | 3 |

**Table 3**

| **Summary of Dispersion Compositions for Antislip Coatings G-L** | | | | | | |
|---|---|---|---|---|---|---|
| Component | Parts by Weight in Coating | | | | | |
| | G | H | I | J | K | L |
| Michem® 4983 | 100 | 100 | 100 | 100 | 100 | 100 |
| Expancel® 051WU | 15 | 15 | --- | 5 | 30 | 60 |
| Rosin soap | 2 | --- | --- | --- | --- | --- |
| Potassium oleate | --- | 2 | --- | --- | --- | --- |
| Colorant | 3 | 3 | 3 | 3 | 3 | 3 |

**Table 4**

| **Summary of Dispersion Compositions for Antislip Coatings M-R** | | | | | | |
|---|---|---|---|---|---|---|
| Component | Parts by Weight in Coating | | | | | |
| | M | N | O | P | Q | R |
| Rhoplex® HA16 | --- | --- | --- | 100 | --- | --- |
| Styronal® ND846 | --- | 50 | 100 | --- | --- | --- |
| Rhoplex® B-15 | --- | --- | --- | --- | 100 | --- |
| Styronal® 4574 | 100 | 50 | --- | --- | --- | 100 |
| Expancel® 051WU | 15 | 15 | 15 | --- | --- | --- |
| Colorant | 3 | 3 | 3 | --- | --- | --- |

**Table 5**

| **Summary of Dispersion Compositions for Antislip Coatings S and T** | | |
|---|---|---|
| Component | Parts by Wt. in Coating | |
| | S | T |
| Styronal® ND846 | 50 | 100 |
| Styronal® 4574 | 50 | --- |

Twenty-four different types of sheets were tested, with each type constituting an example. Examples 1-9, inclusive, constituted controls. Example 1 consisted simply of an uncoated sheet. Examples 2 and 3 utilized antislip coatings containing inorganic particles. The remaining controls involved the use of various binders without thermally expandable microbeads being present. Examples 10-24, inclusive, all had an antislip coating coming within the scope of the present invention. Table 6 summarizes the combination of paper and coating composition for each example.

**Table 6**

| **Summary of Paper and Coating Compositions Employed in the Examples** | | |
|---|---|---|
| Example | Paper | Coating |
| 1 | C | --- |
| 2 | A | A |
| 3 | B | B |
| 4 | A | I |
| 5 | C | T |
| 6 | C | P |
| 7 | C | S |
| 8 | C | R |
| 9 | C | Q |
| 10 | C | C |
| 11 | C | C |
| 12 | C | J |
| 13 | C | H |
| 14 | C | K |
| 15 | C | L |
| 16 | C | D |
| 17 | C | E |
| 18 | C | F |
| 19 | C | G |
| 20 | C | O |
| 21 | C | O |
| 22 | C | O |
| 23 | C | N |
| 24 | C | M |

Wet and dry static and dynamic coefficients of friction were determined for the sheets obtained from the examples. It was observed, however, that the curves for the dry dynamic coefficient of friction tests with the sheets of Examples 8, 9, 10, 11, 16, 17, and 24 were noncontinuous. Thus, the dry dynamic values in such instances essentially were averages of a series of static coefficient of friction curves and, as a consequence, may be less reliable. The results are summarized in Table 7.

**Table 7**

| **Coefficient of Friction (COF) Results** | | | | |
|---|---|---|---|---|
| Example | Static COF | | Dynamic COF | |
| | Dry | Wet | Dry | Wet |
| 1 | 0.220 | 1.701 | 0.299 | 1.701 |
| 2 | 0.169 | 0.680 | 0.175 | 0.675 |
| 3 | 0.254 | 0.655 | 0.275 | 0.750 |
| 4 | 0.575 | 1.694 | 0.277 | 1.319 |
| 5 | 0.399 | 0.401 | 0.320 | 0.654 |
| 6 | 0.387 | 0.618 | 0.296 | 0.657 |
| 7 | 0.626 | 1.009 | 0.388 | 1.250 |
| 8 | 3.103 | 2.660 | 0.910 | 1.842 |
| 9 | 2.028 | 1.270 | 1.600 | 1.295 |
| 10 | 1.313 | 0.727 | 1.763 | 0.838 |
| 11 | 1.967 | 0.651 | 2.750 | 0.788 |
| 12 | 1.729 | 1.538 | 2.074 | 1.731 |
| 13 | 1.688 | 1.408 | 2.000 | 1.242 |
| 14 | 1.900 | 1.115 | 2.004 | 1.288 |
| 15 | 1.575 | 1.144 | 1.674 | 1.350 |
| 16 | 0.717 | 1.710 | 1.217 | 1.926 |
| 17 | 1.569 | 1.329 | 1.781 | 1.349 |
| 18 | 1.938 | 0.938 | 2.269 | 0.909 |
| 19 | 1.650 | 1.044 | 1.994 | 1.110 |
| 20 | 1.002 | 1.015 | 1.291 | 1.213 |
| 21 | 0.825 | 1.053 | 1.131 | 1.282 |
| 22 | 0.616 | 1.168 | 0.821 | 1.333 |
| 23 | 1.161 | 1.424 | 1.265 | 1.679 |
| 24 | 1.602 | 2.059 | 1.454 | 1.997 |

In order to aid in understanding and differentiating the coefficient of friction data presented in Table 7, two different calculations were made, i.e., COF_{W} - COF_{D} and COF_{W} + COF_{D}, in which coefficient of friction is abbreviated as COF with the subscript "W" or "D" to represent a wet or dry coefficient of friction, respectively. The results of these calculations are summarized in Tables 8 and 9.

**Table 8**

| **Summary of Calculations with Static Coefficient of Friction Data** | | |
|---|---|---|
| Example | Static COF Calculations | |
| | Wet - Dry | Wet + Dry |
| 1 | 1.48 | 1.92 |
| 2 | 0.51 | 0.85 |
| 3 | 0.40 | 0.91 |
| 4 | 1.12 | 2.27 |
| 5 | 0.00 | 0.80 |
| 6 | 0.23 | 1.00 |
| 7 | 0.38 | 1.64 |
| 8 | -0.44 | 5.76 |
| 9 | -0.76 | 3.30 |
| 10 | -0.59 | 2.04 |
| 11 | -1.32 | 2.62 |
| 12 | -0.19 | 3.27 |
| 13 | -0.28 | 3.10 |
| 14 | -0.78 | 3.02 |
| 15 | -0.43 | 2.72 |
| 16 | 0.99 | 2.43 |
| 17 | -0.24 | 2.90 |
| 18 | -1.00 | 2.88 |
| 19 | -0.61 | 2.69 |
| 20 | 0.01 | 2.02 |
| 21 | 0.23 | 1.88 |
| 22 | 0.55 | 1.78 |
| 23 | 0.26 | 2.58 |
| 24 | 0.46 | 3.66 |

**Table 9**

| **Summary of Calculations with Dynamic Coefficient of Friction Data** | | |
|---|---|---|
| Example | Dynamic COF Calculations | |
| | Wet - Dry | Wet + Dry |
| 1 | 1.40 | 2.00 |
| 2 | 0.50 | 0.85 |
| 3 | 0.48 | 1.02 |
| 4 | 1.04 | 1.60 |
| 5 | 0.33 | 0.97 |
| 6 | 0.36 | 0.95 |
| 7 | 0.86 | 1.64 |
| 8 | 0.93 | 2.75 |
| 9 | -0.30 | 2.90 |
| 10 | -0.92 | 2.60 |
| 11 | -1.96 | 3.54 |
| 12 | -0.34 | 3.80 |
| 13 | -0.76 | 3.24 |
| 14 | -0.72 | 3.29 |
| 15 | -0.32 | 3.02 |
| 16 | 0.71 | 3.14 |
| 17 | -0.43 | 3.13 |
| 18 | -1.36 | 3.18 |
| 19 | -0.88 | 3.10 |
| 20 | -0.08 | 2.50 |
| 21 | 0.15 | 2.41 |
| 22 | 0.51 | 2.15 |
| 23 | 0.41 | 2.94 |
| 24 | 0.54 | 3.45 |

Based on the data in Table 8, several conclusions are possible. First, the difference between the wet and dry static coefficients of friction, i.e., COF_{W} - COF_{D}, preferably is in the range of from about - 1.5 to about 1. Second, the sum of the wet and dry static coefficients of friction, i.e., COF_{W} + COF_{D}, preferably is in the range of from about 1.7 to about 3.8. Second, if the difference between the wet and dry static coefficients of friction, i.e., COF_{W} - COF_{D}, is less than -0.4 and greater than -1.0, then the sum of the wet and dry static coefficients of friction, i.e., COF_{W} + COF_{D}, preferably is equal to or less than about 3.0. Third, if the difference between the wet and dry static coefficients of friction, i.e., COF_{W} - COF_{D}, is greater than 0 and less than 1.0, then the sum of the wet and dry static coefficients of friction, i.e., COF_{W} + COF_{D}, preferably is greater than about 1.7

### Example 25

### Application of Coating to Dispensing Container

The paper of Example 11 was applied to bottom surface of a dispenser-size, Pop-Up® Space-Saver® Signals® Box of Kleenex® Brand facial tissues. The bottom of the container measured 12.1 by 24.1 cm (4.75 by 9.5 inches). The paper of Example 11 was cut into two strips, each of which was 3.8 cm (1.5 inches) wide and 14 cm (5.5 inches) long. The strips were affixed with rubber cement along the front and rear edges of the bottom surface of the Kleenex® Box. Each strip was centered on each edge, with the front edge of the front strip being contiguous with the front edge of the bottom surface. Similarly, the rear edge of the rear strip was contiguous with the rear edge of the bottom surface.
The Kleenex^{R} Box exhibited a significantly reduced tendency to slip during use when placed on a polished wood surface.

## Claims

1. An article of manufacture comprising a substrate having a smooth nonabrasive antislip coating on part or all of a surface thereof, which coating:
A. is comprised of from 80 to 99 percent by weight, based on the weight of the coating, of a binder having a glass transition temperature no lower than about -30°C and from 1 to 20 percent by weight, based on the weight of the coating, of thermally expanded microbeads having particle sizes before expansion in the range of from 5 to 30 µm;
B. has a dry static coefficient of friction, when tested in accordance with ASTM Method 1894 against an aluminum plate having an anodized surface with a roughness of less than about 0.8 µm, of at least about 0.6; and
C. has a dry dynamic coefficient of friction, when tested in accordance with ASTM Method 1894 against an aluminum plate having an anodized surface with a roughness of less than about 0.8 µm, of at least about 0.8;
in which said substrate is preferably selected from the group consisting of papers, films, and woven and nonwoven fabrics.

2. The article of claim 1, in which said coating:
A. has a wet static coefficient of friction, when tested in accordance with ASTM Method 1894 against an aluminum plate having an anodized surface with a roughness of less than about 0.8 µm, of at least about 0.6; and
B. has a wet dynamic coefficient of friction, when tested in accordance with ASTM Method 1894 against an aluminum plate having an anodized surface with a roughness of less than about 0.8 µm, of at least about 0.7.

3. The article of claim 1 or 2, in which said binder has a glass transition temperature no lower than about 15°C.

4. The article of any one of the preceding claims, in which said binder has a glass transition temperature no lower than about 25°C.

5. The article of any one of the preceding claims, in which the static coefficients of friction of said coating are such that COF_{W} - COF_{D} is in the range of from -1.5 to 1.

6. The article of claim 5, in which the static coefficients of friction of said coating are such that, if COF_{W} - COF_{D} is less than -0.4 and greater than -1.0, then COF_{W} + COF_{D} is equal to or less than about 3.0.

7. The article of claim 5, in which the static coefficients of friction of said coating are such that, if COF_{W} - COF_{D} is greater than 0 and less than 1.0, then COF_{W} + COF_{D} is greater than about 1.7

8. The article of any one of the preceding claims, in which the dynamic coefficients of friction of said coating are such that COF_{W} + COF_{D} is in the range of from 1.7 to 3.8.

9. The article of any one of the preceding claims in which said binder is a latex.

10. The article of any one of the preceding claims, in which said article is a surgical drape having said coating on at least a portion of the bodyside layer of said drape.

11. The article of claim 10, in which said surgical drape comprises a laminate of two or more layers of nonwoven webs.

12. The article of any one of claims 1 to 9, in which said article is a shoe cover having said coating on at least a portion of the outer sole thereof.

13. The article of any one of claims 1 to 9 which comprises as substrate a paper having a first surface and said second surface, in which said first surface has said smooth nonabrasive, antislip coating adjacent to and contiguous with said first surface.

14. The article of claim 13, in which said paper is a latex-impregnated paper.

15. The article of claim 14, in which said second surface has a barrier coating adjacent thereto and contiguous therewith.

16. The article of claim 15, in which said barrier coating has an abrasive coating adjacent thereto and contiguous therewith.

17. The article of any one of claims 1 to 9 wherein the article is a dispensing container having the smooth nonabrasive antislip coating on part or all of the bottom surface thereof.
